# EUROPEAN PATENT APPLICATION

(11) **EP 1 659 116 A1**
(43) Date of publication of application: **24.05.2006**
(21) Application number: 03748519.0
(22) Date of filing: 29.08.2003
(51) Int. Cl.: C07D 273/00, C12N 1/14, C12P 17/14, A61K 31/395, A61P 31/10

(54) **FKI-1366 SUBSTANCES AND PROCESS FOR PRODUCING THE SAME**

(71) Applicant: THE KITASATO INSTITUTE, Tokyo 108-8642 (JP)
(72) Inventor: OMURA, Satoshi, Setagaya-ku, Tokyo 157-0076 (JP); TOMODA, Hiroshi, Chofu-shi, Tokyo 182-0034 (JP); MASUMA, Rokuro, Minato-ku, Tokyo 108-0073 (JP); Arai, Masayoshi, Soka-shi, Saitama 340-0023 (JP)
(74) Representative: Duckworth, Timothy John
(86) International application number: PCT/JP2003/011053
(87) International publication number: WO 2005/023788

(57) **Abstract**

A microorganism belonging to Beauveria sp. and having ability to produce FKI-1366 substance A and/or FKI-1366 substance B and/or FKI-1366 substance C (Beauveria sp. FKI-1366 FERM BP-08459) is cultured in a medium, accumulating FKI-1366 substance A and/or FKI-1366 substance B and/or FKI-1366 substance C in the culture liquid and isolating FKI-1366 substance A and/or FKI-1366 substance B and/or FKI-1366 substance C from said cultured mass. Since the thus obtained FKI-1366 substance A and/or FKI-1366 substance B and/or FKI-1366 substance C or composition thereof have activity to enhance azole antifungal agent, the substance has an action against various fungal infections such as deep-seated mycosis and other fungal infections in the low concentration within short term. Consequently, the substance is useful for reducing frequency of appearance of resistant microorganisms and overcoming resistance, and is expected as a medicament.

## Description

### Technical field

The present invention relates to novel FKI-1366 substance having potentiation activity for azole antifungal agents and production thereof. FKI-1366 substance is a general term for FKI-1366 A substance, FKI-1366 B substance and FKI-1366 C substance.

### Prior arts

With regard to azoles used for treatment of mycosis, 1-[(2-(2,4-dichlorobenzyloxy)-2-(2-,4-dichlorophenyl )ethyl] imidazole (generic name: miconazole, Sigma Inc., U.S.),
2,4-difluoro-α,α-bis(1H-1,2,4-triazol-1-yl-methyl) benzyl alcohol (generic name: fluconazole, ICN Pharmaceuticals Inc., U.S.) and (±)-1-sec-butyl-4-[p-[4-[p-[[(2R,4S)-2-(2,4-dichloro phenyl)-2-(1H-1,2,4-triazol-1-yl-methyl)-1,3-dioxola n-4-yl]methoxy]phenyl]-piperazinyl]phenyl]-Δ²-1,2,4-triazolin-5-one (genericname: itraconazole, Kyowa Hakko Kogyo K. K., Japan), are known.

These compounds are highly safety as compared with polyene antibiotics used for treatment of mycosis, e.g. (1R, 3S, 5R, 6R, 9R, 11R, 15S, 16R, 17R, 18S, 19E, 21E, 23E, 25E, 27E, 29E, 31E, 33R, 35S, 36S, 37S-33-(3-amino-3,6-dideoxy-b-D-manopyraosyloxy)-1,3 ,5,6,9,11,17,37-octahydroxy-15,16,18-trimethyl-13-ox o-14,39-dioxabicyclo[33.3.1]nonatriaconta-19,21,23,2 5,27,29,31-heptanene-36-carboxylic acid (generic name: amphotericin B, Sigma Inc., U.S.), and are most frequently used drugs (Anaissie E. J. et al., Clinical Infectious Diseases, 23: 964-972, 1966).

However, recently, appearance of resistant microbes caused by long-term or repetitive administration of these azole antifungal agents becomes problem. As a result, development of drugs with high safety and low emergence rate of resistance microorganisms is urgently necessary.

Main stream of drug development of antifungal agents up to this time is the development of drugs directly acting against fungi with fungicidal or fungistatic action. Creation of pharmaceuticals has never been performed from the viewpoint of potentiation of activity of known antifungal agents

### Disclosure of the invention

In diseases accompanied with immunocompromised conditions such as HIV infection and hematologic disease, the compromisedhost condition is induced, and a frequency of generation of fungal infection is increased as an opportunistic infection. Diseases with these immunocompromised conditions are frequently serious, and long-term therapy is required. Consequently, at present the most frequently used azole antifungal agents may be thought in a condition of inducing drug resistance, since the long term chemotherapy of fungal infection is required in many cases.

A mechanism of resistance against azole antifungal agents is known as follows. In Candida albicans, a target enzyme, P-450, i.e. excess expression of 14-α-demethylase or decrease in affinity with drug as a result of amino acid mutation (Vanden Bossche, H. et al. Antimicrobial Agents and Chemotherapy, 36, 2602-2610, 1992; Sanglard, D. et al., ibid., 42, 241-253, 1998) and decrease in intracellular drug concentration by the multiple drug excretion transporter such as MSF (major facilitator Superfamily) and ABC (ATP binding cassette) (Fing, M. E. et al., Mol. Gen. Gent., 227, 318-329, 1991; Sanglard, D. et al., Microbiology, 143, 405-416, 1997) are known. In Saccharomyces cerevisiae, it has reported thatMDR (MulltipleDrugResistant) gene, PDR16 and PDR17, could change the lipid metabolism to acquire the resistance against azole compounds (H. Bart van den Hazel, et al., J. Biol. Chem., 274, 1934-1941, 1999).

Consequently, the drugs, which can increase activity of azole antifungal agents, may be expected to decrease the frequency of appearance of the resistant microorganisms by reducing amount of administration of drug as well as shortening the administration term. Further, as a result of use in combination with drugs having two different skeletal structures, it can be expected to be effective for the resistance against azole antifungal agent. In such condition, to provide drugs having enhanced activity of azole antifungal agents is useful for treatment of various fungal infections such as deep-seated mycosis and azole resistant mycosis. Means for solving problems

An aspect of the present invention is to provide FKI-1366 substance, which is a general term for FKI-1366 substance A, FKI-1366 substance B and FKI-1366 substance C, having enhancing activity for practically used antifungal agents at present, and having enhancing activity for antifungal agents, which can act against variety of mycosis such as deep-seated mycosis at low concentration within short time and make them possible to lower frequency of appearance of resistant fungal strain, and a production thereof.

We have found that, as a result of studies on metabolites produced by various microorganisms, the substance having properties to activate azole antifungal agents was produced in a cultured mass of the strain FKI-1366 which was newly isolated from soil. We have also found, as a result of isolating and purifying the active substance from the cultures mass, substances having the chemical structure represented by the formula [I], [II] and [III] hereinbelow. Since the substances having the chemical structure represented by the formula [I], [II] and [III] have not known so far and were designated as FKI-1366 substance A, FKI-1366 substance B and FKI-1366 substance C.

The present invention was completed as a result of these findings. An object of the present invention is to provide FKI-1366 substance A represented by the chemical structure [I] hereinbelow.

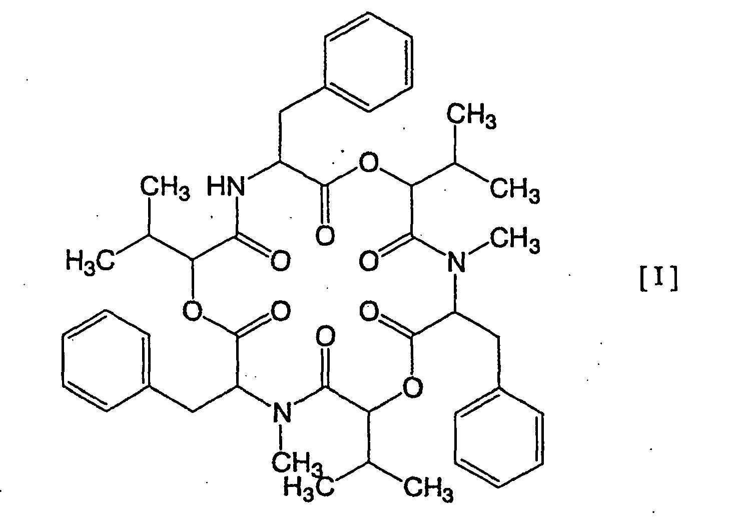

Another object of the present invention is to provide FKI-1366 substance B represented by the chemical structure [II] hereinbelow.

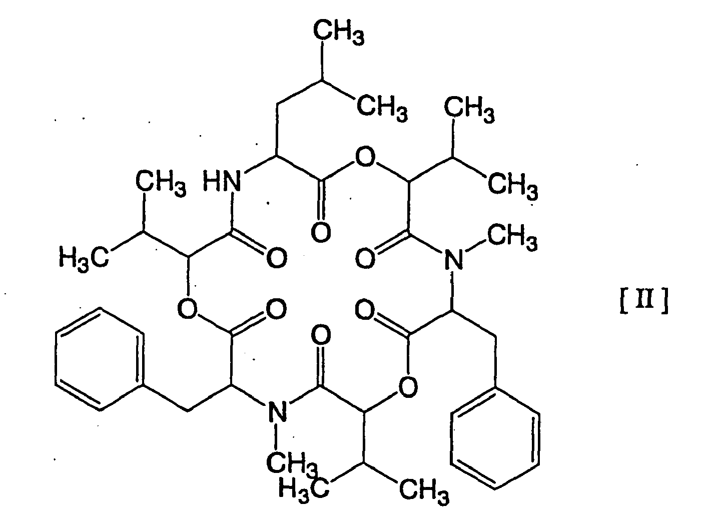

Further object of the present invention is to provide FKI-1366 substance C represented by the chemical structure [III] hereinbelow.

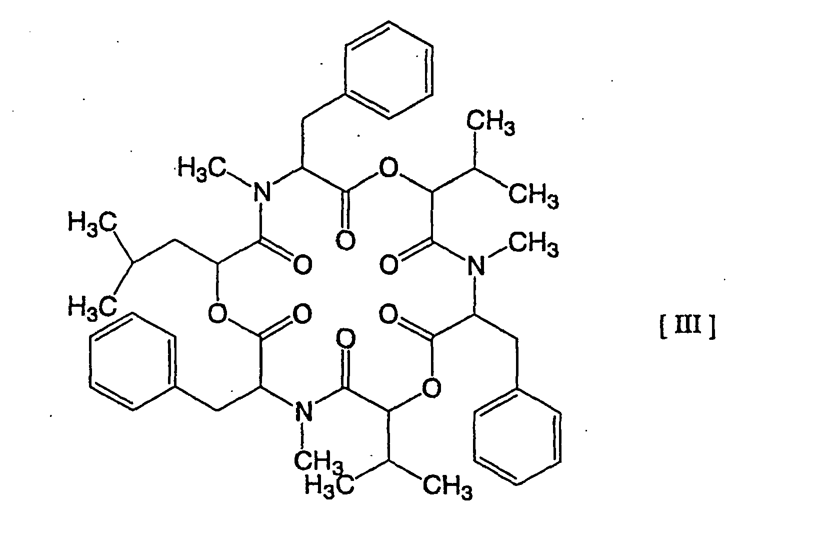

Further object of the present invention is to provide crude FKI-1366 substance comprising FKI-1366 substance A and/or FKI-1366 substance B and/or FKI-1366 substance C represented by the chemical structure [I], [II] and [III], respectively, hereinabove.

More further object of the present invention is to provide a process for production of FKI-1366 substance A comprising culturing a microorganism belonging to Beauveria sp. having ability to produce FKI-1366 substance A in a medium, accumulating FKI-1366 substance A in a culture liquid and isolating FKI-1366 substance A from the cultured mass.

Further object of the present invention is to provide a process for production of FKI-1366 substance B comprising culturing a microorganism belonging to Beauveria sp. having ability to produce FKI-1366 substance B in a medium, accumulating FKI-1366 substance B in a culture liquid and isolating FKI-1366 substance B from the cultured mass.

Further object of the present invention is to provide a process for production of FKI-1366 substance C comprising culturing a microorganism belonging to Beauveria sp. having ability to produce FKI-1366 substance C in a medium, accumulating FKI-1366 substance C in a culture liquid and isolating FKI-1366 substance C from the cultured mass.

Further object of the present invention is to provide a process for production of crude FKI-1366 substance comprising culturing a microorganism belonging to Beauveria sp. having ability to produce FKI-1366 substance A and/or FKI-1366 substance B and/or FKI-1366 substance C in a medium, accumulating FKI-1366 substance A and/or FKI-1366 substance B and/or FKI-1366 substance C in a culture liquid and isolating FKI-1366 substance A and/or FKI-1366 substance B and/or FKI-1366 substance C from the cultured mass.

Further object of the present invention is to provide the process for production of crude FKI-1366 substance wherein the microorganism having ability to produce FKI-1366 substance A and/or FKI-1366 substance B and/or FKI-1366 substance C is Beauveria sp. FKI-1366 FERM BP-08459.

Further object of the present invention is to provide the microorganism belonging to Beauveria sp. and having ability to produce FKI-1366 substance A, FKI-1366 substance B or FKI-1366 substance C.

Further object of the present invention is to provide the microorganism Beauveria sp. FKI-1366 FERM BP-08459.

The microorganism having ability to produce FKI-1366 substance A or FKI-1366 substance B or FKI-1366 substance C represented by the formula [I], [II] or [III] hereinbefore (hereinafter designates as "FKI-1366 substance producing microorganism") belongs to Beauveria sp. , however the microorganism is not limited to the above and can be the strain, without limitation, having ability to produce the substance of the present invention. A preferable example of the strain used for production of FKI-1366 substance of the present invention is for example Beauveria sp. FKI-1366 which was newly isolated from the soil sample by the present inventors.

Taxonomical properties of the strain are shown as follows.

### (1) Morphological properties:

Good growth is observed in potato-dextrose agar medium, malt extract agar medium, corn meal agar medium and Miura' s agarmedium. Bearing conidia is good in various agar media.

A microscopic observation of colonies grown on Miura' s agar medium indicates colorless hyphae with septa, and conidiogenous organs, singular or clustering, directly standing from aerial mycelia or from short branches (2.8 - 4.3 × 1.8 - 2.8 µm). Basements of conidiogenous organs are bulging to subspherical or globose with 3.3 - 5.6 × 2.0 - 3.3 µm in size. A top of conidiogenous organs elongates sharply to zigzag form depending on forming conidia with length 5 - 12 µm. Conidia with globose to subglobose colorless form, 1.8 - 3.0 × 1.8 - 2.6 µm, are formed with budding on the small protrusive region of the conidiogenous organ of the conidium. Aerial mycelia bearing rod shaped or irregularly branched chlamydospore are observed.

### (2) Cultured properties on various agar medium

Results of macroscopic observation cultured on various agar media at 25°C for 14 days are shown in the following Table.

| Medium Growth on medium (diameter of colony) | Color tone of surface of colony | Color tone of reverse of colony | Soluble pigment |
|---|---|---|---|
| Potato-dextrose agar medium good (38 - 40 mm) floccose, raised, smooth penumbra | White | pale yellowish brown | none |
| Malt extract agar medium Good (41 - 42 mm) floccose - powdery, raised, penumbra irregular | White | pale yellowish brown | none |
| Corn meal agar medium good (51 - 52 mm) floccose, centrally raised, smooth penumbra | White | white - pale yellowish brown | none |
| Miura's agar medium Good (69 - 70 mm) | White | white - pale yellowish | none |
| floccose - powdery, smooth penumbra | | brown | |

### 3. Physiological properties

### 1) Optimum growth condition

Optimum growth condition of the strain is pH 3. 4-7. 9 at 13.2 - 30.5°C.

### 2) Growth range:

Growth range of the strain is pH 2.4 - 9.8 at 7.4 - 32.4°C.

### 3) Differentiation of aerobic or anaerobic: aerobic.

As described hereinabove, as a result of comparative studies based on the morphological properties, culture properties and physiological properties of the strain FKI-1366 with the known strains, this strain was identified as a strain belonging to genus Beauveria and referred as Beauveria sp. FKI-1366. This strain was deposited with the name of Beauveria sp. FKI-1366 in the International Patent Organism Depository, National Institute of Advanced Industrial Science and Technology, Higashi 1-1-3, Tsukuba-city, Ibaragi-ken, Japan and given a permanent depository No. FERM BP-08459 on August 27, 2003.

Example of FKI-1366 substance A, FKI-1366 substance B and FKI-1366 substance C producing strain used in the present invention is the aforesaidBeauveria sp. FKI-1366, but as a general nature of the microorganisms, the strain is easily mutated and not constantly maintained in its nature, and it is well known to mutate naturally or artificially by using ultraviolet irradiation, X-ray irradiation or treatment with mutagenic agents such as N-methyl-N'-nitro-N-nitrosoguanidine or 2-aminopurine. The strains belonging to Beauveria sp. including artificial mutants, cell fusion strain and genetically engineered strain and producing FKI-1366 substance A, FKI-1366 substance B and FKI-1366 substance C can be used in the present invention.

In the production of FKI-1366 substance A, FKI-1366 substance B and FKI-1366 substance C of the present invention, FKI-1366 substance A, FKI-1366 substance B and FKI-1366 substance C producing strain belonging to Beauveria sp. is cultured in the preferable medium. Examples of preferable nutrient sources for production of FKI-1366 substance A, FKI-1366 substance B and FKI-1366 substance C are assimilable carbon sources, digestible nitrogen sources, and if necessary inorganic salts, vitamin, etc.

The above described assimilable carbon sources are saccharides such as glucose, fructose, maltose, lactose, galactose, dextrin and starch, and vegetable oil such as soybean oil, and are used alone or in combination.

Examples of digestible nitrogen sources are peptone, yeast extract, meat extract, soybean powder, cotton seed oil, corn steep liquor, malt extract, casein, amino acids, urea, ammonium salt or nitrate and these are used alone or in combination. If necessary, salts such as phosphate, magnesium salt, calcium salt, sodium salt or potassium salt, and heavy metallic salt such as iron salt, manganese salt, copper salt, cobalt salt and zinc salt, various vitamins and substance preferable for production of FKI-1366 substance A, FKI-1366 substance B and FKI-1366 substance C are preferably added.

In the culture, if necessary, antifoam agent such as animal oil, vegetable oil and silicone oil can be added in case of foaming. The above culture can be a liquid culture or solid culture, if the above nutrient sources are contained. Conventionally, it is preferable to use the liquid culture. In case of small-scale culture, the culture using a flask is preferable. In the large scale industrial production, the submerged aeration culture as like in the other fermentation production is preferable.

In case of the large scale culture in the large tank, in order to prevent growth delay of microorganisms in the production process, at first, the strain for production is inoculated into the comparatively small scale medium, subsequently the cultured mass is transferred into the large tank and cultured for mass production. In this case, composition of medium of the former culture and the composition of the medium used in the production scale can be identical with each other or, if necessary, can be changed.

In case of aeration culture with stirring, conventionally known method can be applied, for example, agitation by propeller and other machinery means, rotation or shaking of the fermentor, pumping treatment and blowing aeration. Aeration is sterilized before use.

Culturing temperature can be changed within the range of production of FKI-1366 substance A, FKI-1366 substance B and FKI-1366 substance C by the FKI-1366 substance A, FKI-1366 substance B and FKI-1366 substance C producing temperature and is 20 - 30°C, preferably around 27°C. Culturing pH is usually pH 5 - 8, preferably neutral pH 7. Culturing time is different depending on the culture condition and is generally around 4 - 7 days.

The thus obtained FKI-1366 substance A, FKI-1366 substance B or FKI-1366 substance C exist in the cultured mycelia and culture filtrate. In order to isolate the FKI-1366 substance A, FKI-1366 substance B or FKI-1366 substance C from the cultured mass, whole cultured mass is extracted with water miscible organic solvent such as acetone, and the solvent is distilled off in vacuo, subsequently the residue is extracted with water immiscible organic solvent such as ethyl acetate.

In addition to the above extracting method, known method used for isolation of lipophilic substance, for example, absorption chromatography, gel filtration chromatography, thin-layer chromatography, centrifugal counter-current chromatography and high performance liquid chromatography are preferably combined or repeatedly performed to isolate each component and purified.

The physicochemical properties of FKI-1366 substance A, FKI-1366 substance B or FKI-1366 substance C of the present invention are as follows.

### FKI-1366 substance A

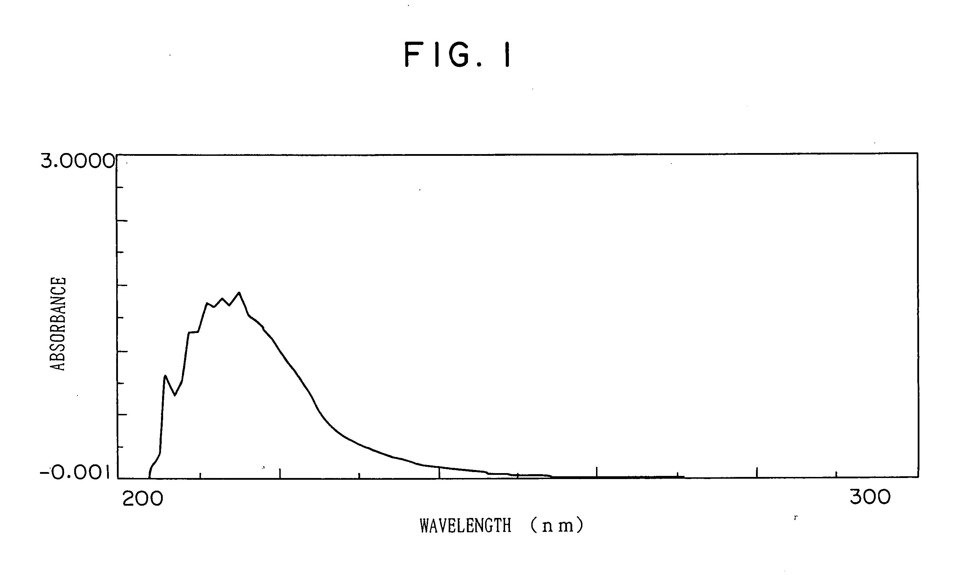

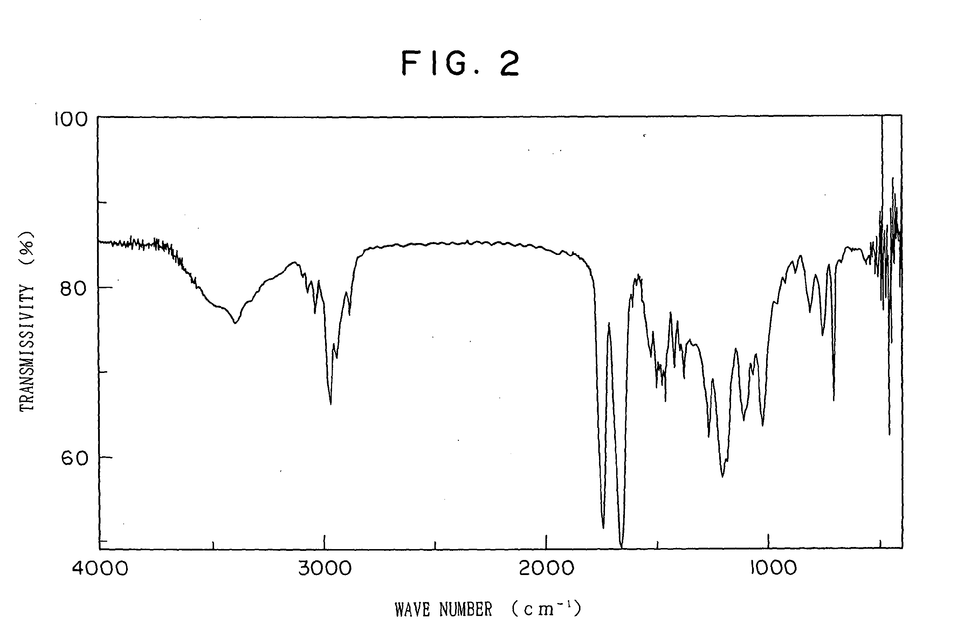

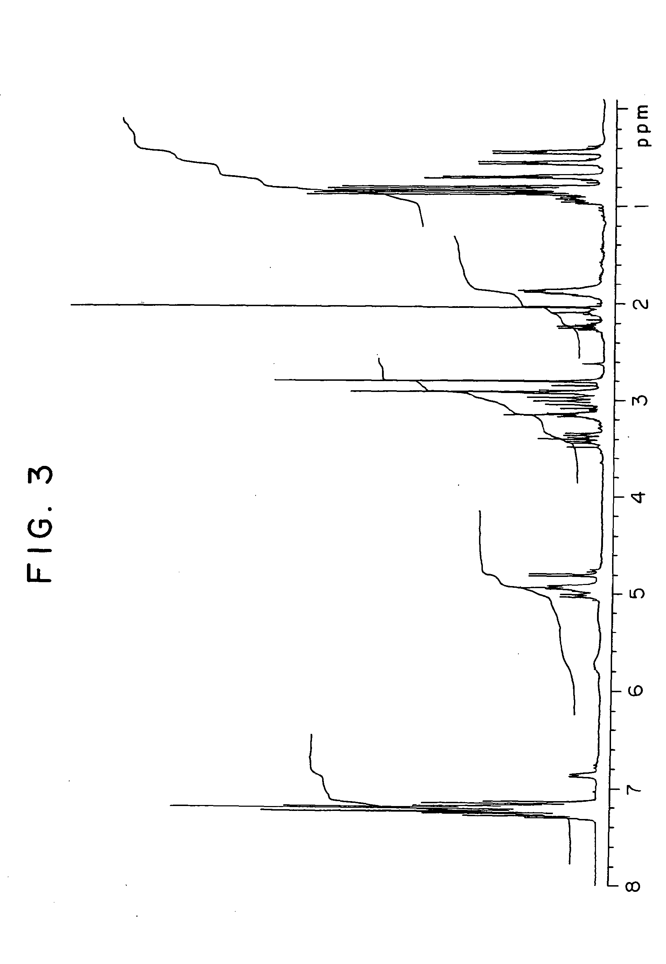

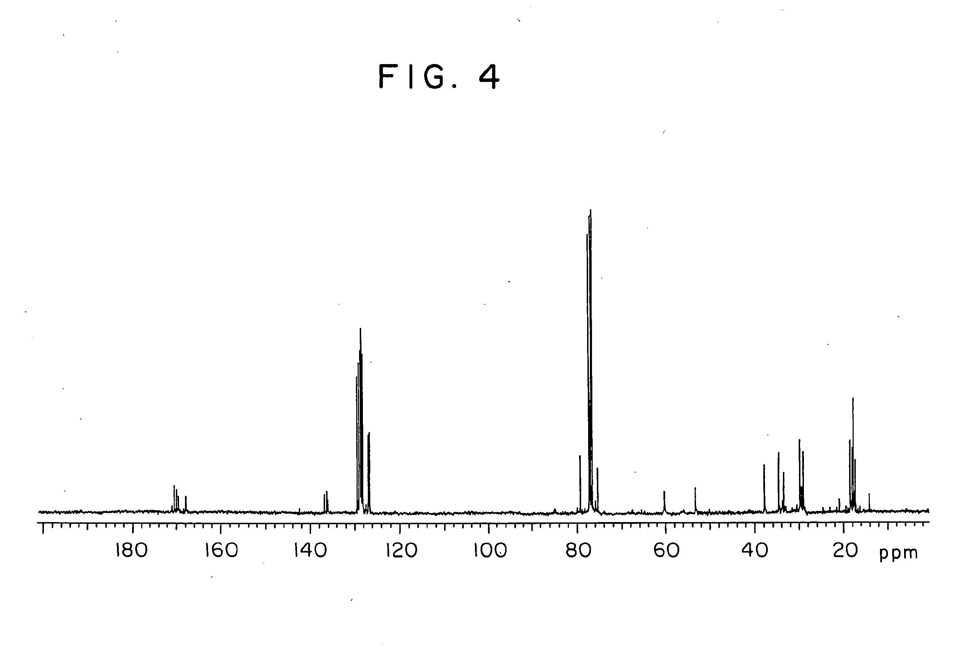

(1) Nature: white powder.
(2) Molecular weight: 769 (fast atom bombardment mass spectrometry)
(3) Molecular formula: C₄₄H₅₅O₉N₃
(4) Optical rotation: [α]_{D}²⁶ = +9.0 (C = 0.1, methanol)
(5) UV absorption spectrum: Ultraviolet absorption spectrum measured in methanol is as shown in Fig. 1, and has specific absorption maximum approximately at 213 nm (ε= 26000).
(6) IR absorption spectrum: Infrared absorption spectrum measured in KBr tablet is as shown in Fig. 2, and has specific absorption bands at 2966, 2875, 1743, 1662, 1456, 1261, 1203 and 1105 cm⁻¹.
(7) ¹H-NMR spectrum: As shown in Fig. 3 (measured by using NRM spectrometer, Varian Japan, in CDCl₃).
(8) ¹³C-NMR spectrum: As shown in Fig. 4 (measured by using NRM spectrometer, Varian Japan, in CDCl₃).
(9) Solubility in solvents: Soluble in DMSO, methanol, chloroform and ethyl acetate.
   Slightly soluble in water and n-hexane.
(10) Color reaction: Positive in phosphomolybdic acid
(11) Differentiation in acidic, neutral and alkaline nature: Neutral substance.

As a result of careful examination of the above various physicochemical properties and spectral data of FKI-1366 substance A, the present FKI-1366 substance A was determined to have the following chemical structure [I].

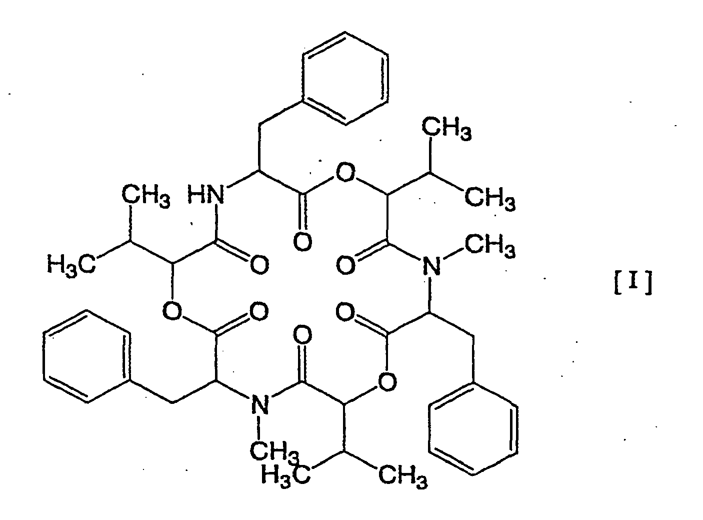

### FKI-1366 substance B

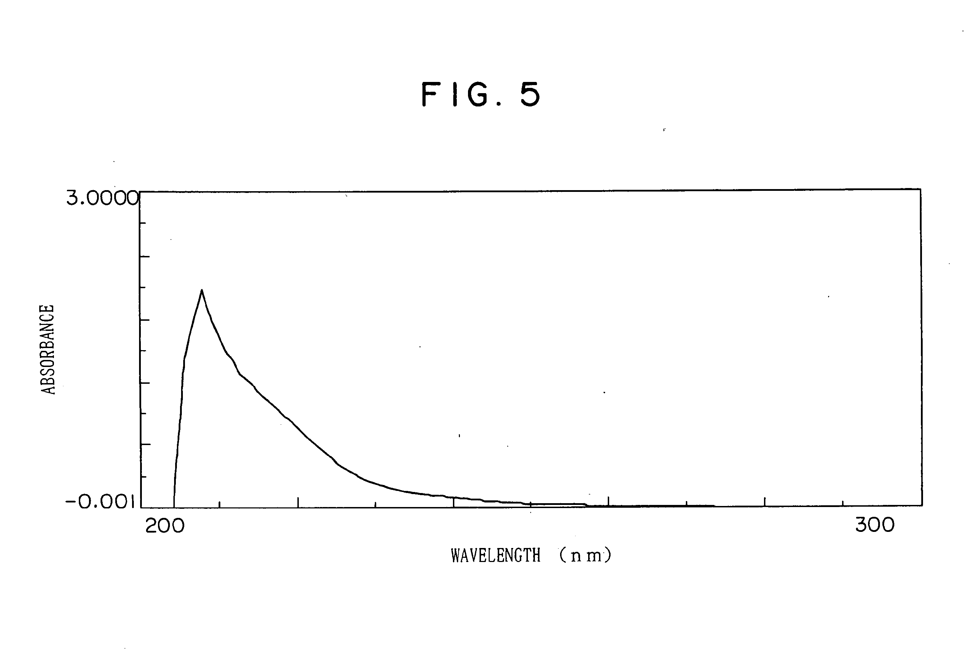

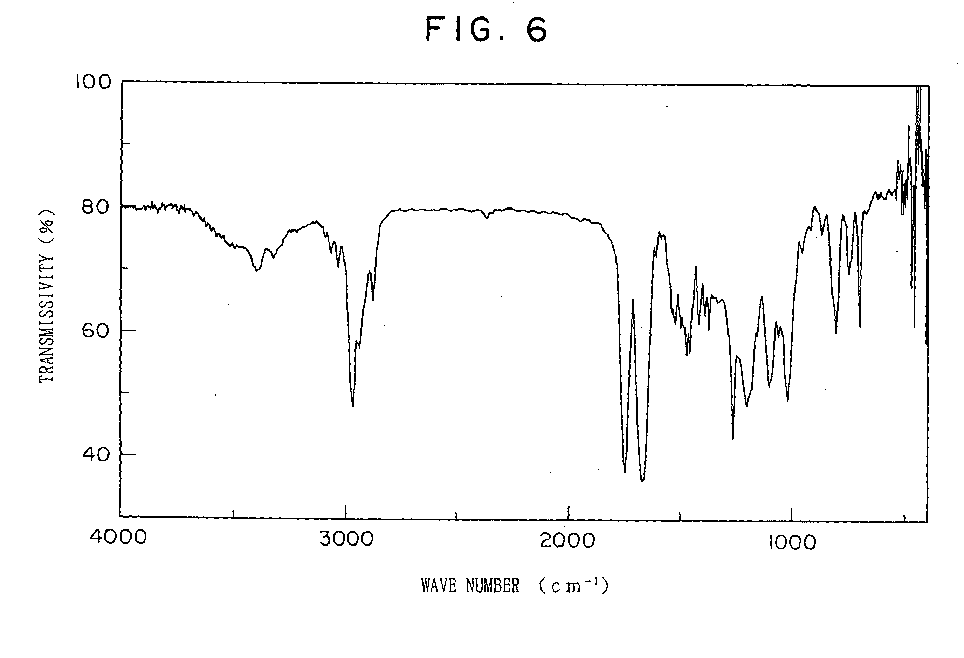

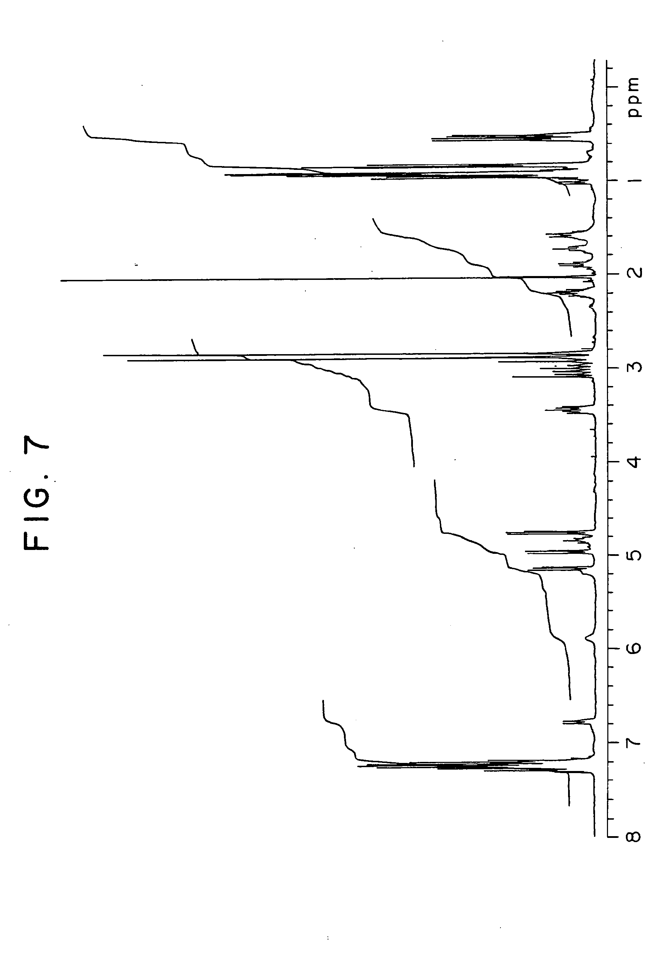

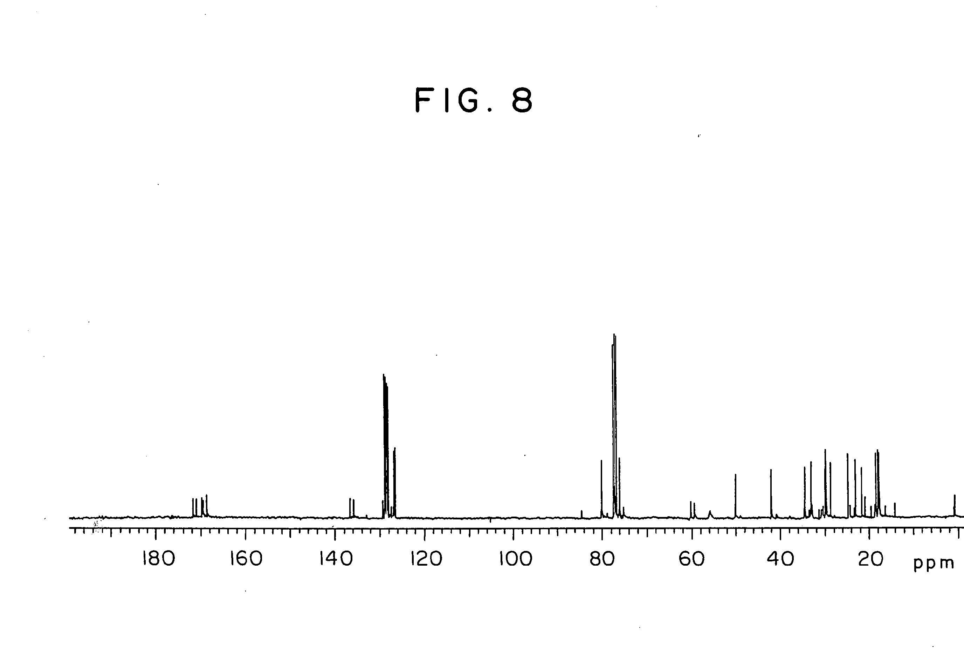

(1) Nature: white powder.
(2) Molecular weight: 735 (fast atom bombardment mass spectrometry)
(3) Molecular formula: C₄₁H₅₇O₉N₃
(4) Optical rotation: [α]_{D}²⁶ = +18.4 (C = 0.1, methanol)
(5) UV absorption spectrum: Ultraviolet absorption spectrum measured in methanol is as shown in Fig. 5, and has specific absorption maximum approximately at 208 nm (ε= 30500).
(6) IR absorption spectrum: Infrared absorption spectrum measured in KBr tablet is as shown in Fig. 6, and has specific absorptionbands at 2964, 2873, 1743, 1664, 1456, 1261, 1201 and 1101 cm⁻¹.
(7) ¹H-NMR spectrum: As shown in Fig. 7 (measured by using NRM spectrometer, Varian Japan, in CDCl₃).
(8) ¹³C-NMR spectrum: As shown in Fig. 8 (measured by using NRM spectrometer, Varian Japan, in CDCl₃).
(9) Solubility in solvents: Soluble in DMSO, methanol, chloroform and ethyl acetate.
   Slightly soluble in water and n-hexane.
(10) Color reaction: Positive in phosphomolybdic acid
(11) Differentiation in acidic, neutral and alkaline nature: Neutral substance.

As a result of careful examination of the above various physicochemical properties and spectral data of FKI-1366 substance B, the present FKI-1366 substance B was determined to have the following chemical structure [II].

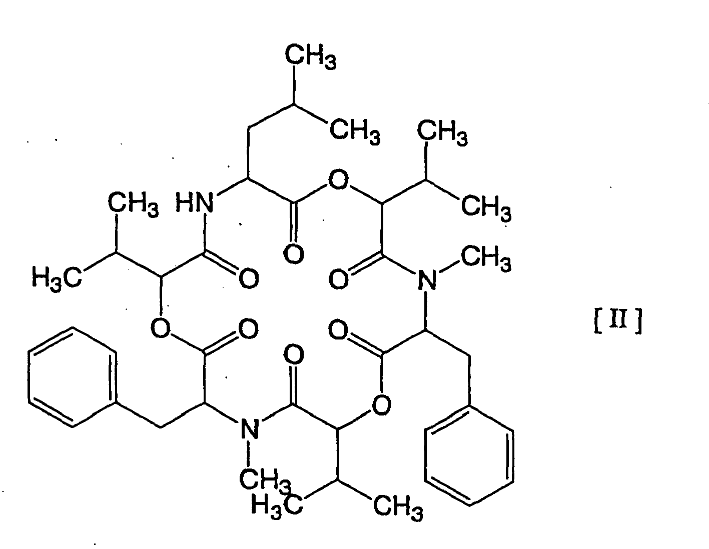

### FKI-1366 substance C

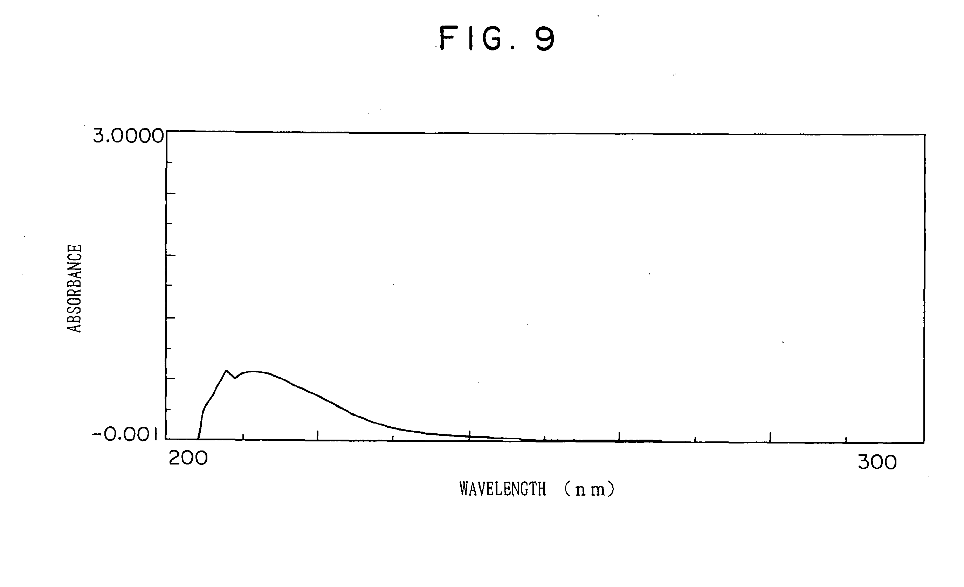

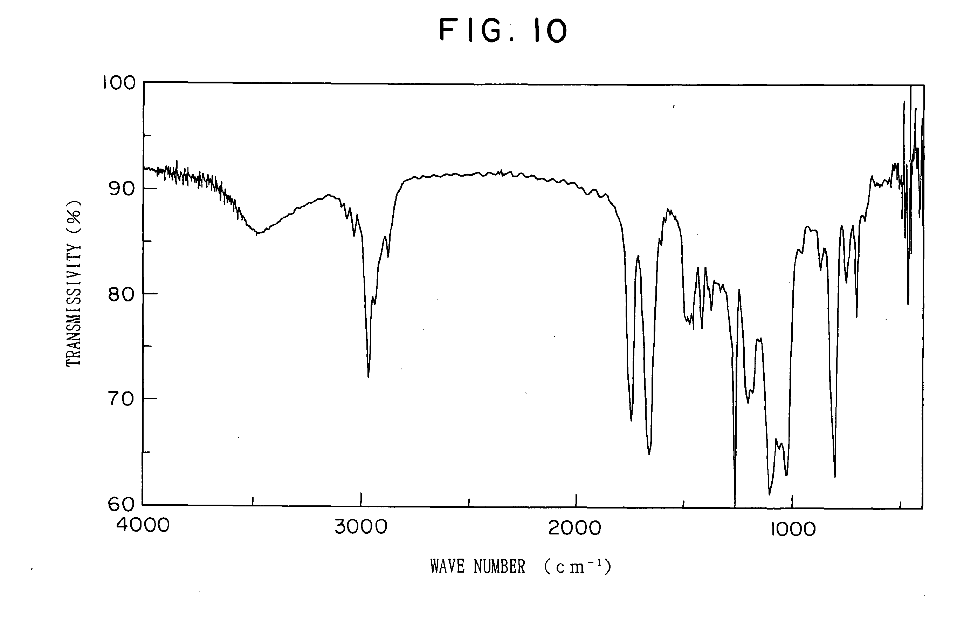

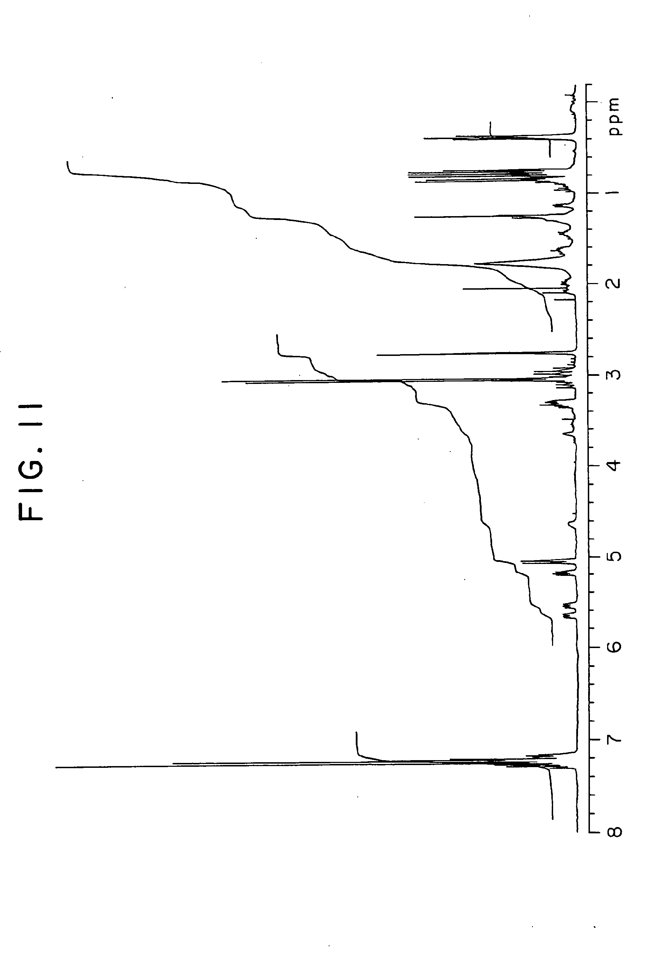

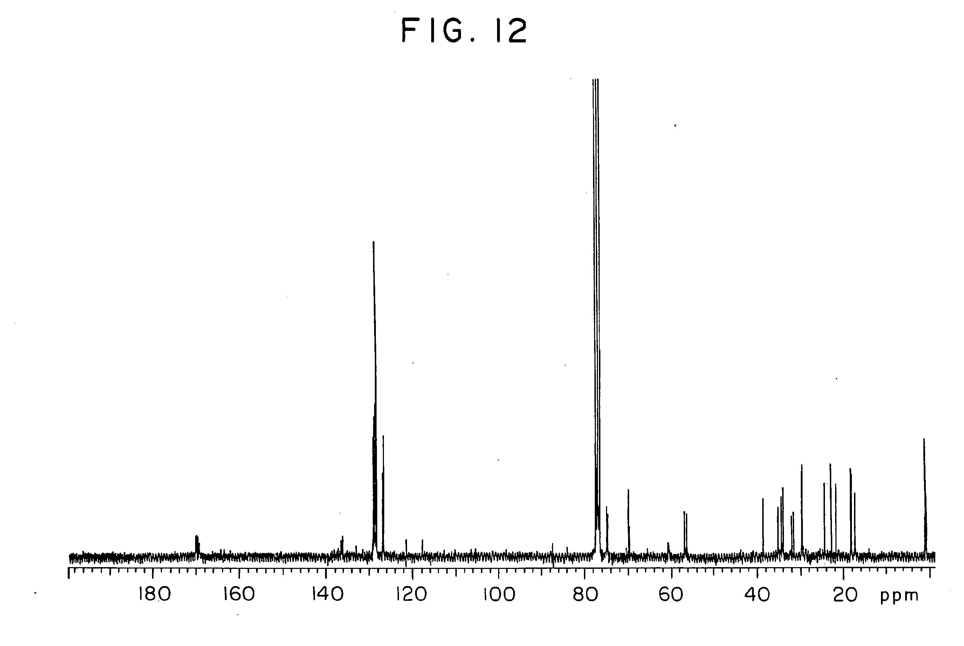

(1) Nature: white powder.
(2) Molecular weight: 797 (fast atom bombardment mass spectrometry)
(3) Molecular formula: C₄₆H₅₉O₉N₃
(4) Optical rotation: [α]_{D}²⁶ = +31.4 (C = 0.1, methanol)
(5) UV absorption spectrum: Ultraviolet absorption spectrum measured in methanol is as shown in Fig. 9, and has specific absorption maximum approximately at 208 nm (ε= 11200).
(6) IR absorption spectrum: Infrared absorption spectrum measured in KBr tablet is as shown in Fig. 10, and has specific absorptionbands at 2962, 2873, 1743, 1662, 1484, 1261, 1203 and 1101 cm⁻¹.
(7) ¹H-NMR spectrum: As shown in Fig. 11 (measured by using NRM spectrometer, Varian Japan, in CDCl₃).
(8) ¹³C-NMR spectrum: As shown in Fig. 12 (measured by using NRM spectrometer, Varian Japan, in CDCl₃).
(9) Solubility in solvents: Soluble in DMSO, methanol, chloroform and ethyl acetate.
   Slightly soluble in water and n-hexane.
(10) Color reaction: Positive in phosphomolybdic acid
(11) Differentiation in acidic, neutral and alkaline nature: Neutral substance.

As a result of careful examination of the above various physicochemical properties and spectral data of FKI-1366 substance C, the present FKI-1366 substance C was determined to have the following chemical structure [III].

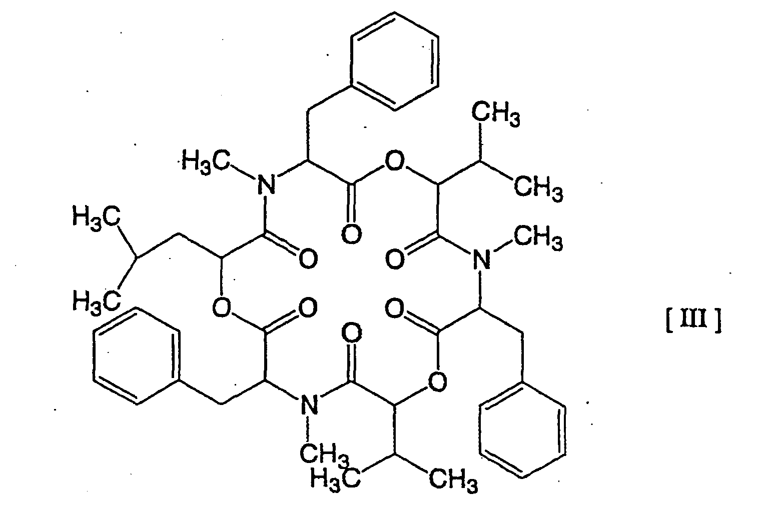

As described hereinabove, the physicochemical properties of FKI-1366 substance A, FKI-1366 substance B or FKI-1366 substance C were described in detail. Compounds having these properties have never been reported previously. Consequently, FKI-1366 substance A, FKI-1366 substance B and FKI-1366 substance C of the present invention are determined as novel substances.

Enhancing activity of FKI-1366 substance A, FKI-1366 substance B and FKI-1366 substance C on azole antifungal agent is described hereinbelow.

Candida albicans ATCC 64548, fluconazole resistant Candida albicans ATCC 64550 and Aspergillus niger ATCC 6275 were used as test organisms. Miconazole and fluconazole were used as azole antifungal agents.

Enhancing activity of azole antifungal agent on Candida albicans ATCC 64548 and ATCC 64550 was determined by yeast susceptibility test method, National Committee for Clinical Standard (NCCLS) Standard Method M27-A. Each suspension of inoculating strain was spread over YM agar medium (0.5% yeast extract, 0.5% malt extract, 0.5% peptone, 1% glucose and 1.5% agar) and cultured at 35°C for 36 hours. Five grown colonies with a diameter 1 mm or more were collected and the colonies were suspended in physiological saline 5 ml. The suspension was further diluted with physiological saline to prepare the test microorganism suspension having McFarland transmittance 0.5. RPMI 1640 medium 70 µl was added into 96 well plastic plate (Corning Inc., U.S.). A solution 20 µl of miconazole or fluconazole, which is prepared for maintaining the final concentration of 0.06 µg/ml - 400 µg/ml, was added thereto. Further, FKI-1366 substance A, FKI-1366 substance B or FKI-1366 substance C, 10 µl, each of which was prepared for maintaining the final concentration of 0.5, 1.0 and 2.0 µg/ml, was added. Finally, the test microorganism suspension 100 µl was added and incubated at 35°C for 24 hours. Absorbancy at 630 nm was measured after 24 hours by using a microplate reader (Elx808, Bio-TEK Instruments Inc., U.S.), and an inhibition concentration 50% (IC₅₀) of azole antifungal agent, which inhibited 50% of growth of each test microorganism under various culture conditions, was calculated. When IC₅₀ value without addition of FKI-1366 substance A, FKI-1366 substance B or FKI-1366 substance C was set as 1.0, a ratio of IC₅₀ with addition of FKI-1366 substance A, FKI-1366 substance B or FKI-1366 substance C was determined as the enhancing activity.

Enhancing activity of azole antifungal agent on Aspergillus niger ATCC 6275 was determined by the drug susceptibility test method for conidiogenous fungi proposed by NCCLS (M38-P). Spores of the strain of Aspergillus niger ATCC 6275 were suspended in the physiological saline to maintain a value at OD 530 nm being 0.09 - 0.11. The suspension was further diluted 100 times with PRMI1640 medium to prepare inoculating microorganism solution. RPMI 1640 medium 70 µl was added into 96 well plastic plate (Corning Inc., U.S.). A solution 20 µl of miconazole or fluconazole, which is prepared for maintaining the final concentration of 1.0 µg/ml - 400 µg/ml, was added thereto. Further, FKI-1366 substance A, FKI-1366 substance B or FKI-1366 substance C, 10 µl, each of which was prepared for maintaining the final concentrationof 2.5, 5.0 and 10 µg/ml, was added. Finally, the test microorganism suspension 80 µl was added. Alamar Blue solution (Asahi Glass Co., Japan) 20µl, a coloring reagent, was added thereto and incubated at 35°C for 24 hours. Fluorescence intensity at excitation wavelength 530 nm and fluorescent wavelength was measured after 24 hours by using Cyto Fluor (Nippon Perceptive Ltd., Japan), and an inhibition concentration 50% (IC₅₀) of azole antifungal agent, which inhibited 50% growth of each test microorganism under various culture conditions, was calculated. When IC₅₀ value without addition of FKI-1366 substance A, FKI-1366 substance B or FKI-1366 substance C was set as 1.0, a ratio of IC₅₀ with addition of FKI-1366 substance A, FKI-1366 substance B or FKI-1366 substance C was determined as the enhancing activity.

Results using miconazole as the azole antifungal agent are shown in Table 1. In Table 1, a symbol * indicates the concentration of FKI-1366 substance A, FKI-1366 substance B or FKI-1366 substance C when A. niger ATCC 6275 was used.

**Table 1**

| C. albicans ATCC64548 IC₅₀(µg/ml) Ratio | | C. albicans ATCC64550 IC₅₀(µg/ml) Ratio | | A. niger ATCC6275 IC₅₀(µg/ml) Ratio | |
|---|---|---|---|---|---|
| Control (miconazole) | | | | | |
| 11.0 | 1.0 | 0.6 | 1.0 | 12.5 | 1.0 |
| +FKI-1366 substance A 0.5 (2.5)* µg/ml | | | | | |
| 11.0 | 1.0 | 0.5 | 1.2 | 2.27 | 5.5 |

| +FKI-1366 substance A 1.0 (5.0)* µg/ml | | | | | |
|---|---|---|---|---|---|
| 7.86 | 1.4 | 0.12 | 5.0 | 1.30 | 9.6 |

| +FKI-1366 substance A 2.0 (10)* µg/ml | | | | | |
|---|---|---|---|---|---|
| <0.2 | >55 | <0.01 | >60 | 1.15 | 10.9 |

| +FKI-1366 substance B 0.5 (2.5)* µg/ml | | | | | |
|---|---|---|---|---|---|
| 6.47 | 1.7 | 0.46 | 1.4 | 6.94 | 1.8 |

| +FKI-1366 substance B 1.0 (5.0)* µg/ml | | | | | |
|---|---|---|---|---|---|
| 1.83 | 6.0 | 0.14 | 4.2 | 5.20 | 2.4 |

| +FKI-1366 substance B 2.0 (10)* µg/ml | | | | | |
|---|---|---|---|---|---|
| <0.2 | >55 | <0.01 | >60 | 3.57 | 3.5 |

| +FKI-1366 substance C 0.5 (2.5)* µg/ml | | | | | |
|---|---|---|---|---|---|
| 11.0 | 1.0 | 0.60 | 1.0 | 2.40 | 5.2 |

| +FKI-1366 substance C 1.0 (5.0)* µg/ml | | | | | |
|---|---|---|---|---|---|
| 4.4 | 2.5 | 0.55 | 1.1 | 1.56 | 8.0 |

| +FKI-1366 substance C 2.0 (10)* µg/ml | | | | | |
|---|---|---|---|---|---|
| <0.2 | >55 | 0.15 | 3.9 | 1.14 | 11.0 |

Results using fluconazole as the azole antifungal agent are shown in Table 2. In Table 1, a symbol * indicates the concentration of FKI-1366 substance A, FKI-1366 substance B or FKI-1366 substance C when A. niger ATCC 6275 was used.

**Table 2**

| | | | | | |
|---|---|---|---|---|---|
| C. albicans | | C. albicans | | A. niger | |

| ATCC64548 IC₅₀(µg/ml) Ratio | | ATCC64550 IC₅₀(µg/ml) Ratio | | ATCC6275 IC₅₀(µg/ml) Ratio | |
|---|---|---|---|---|---|
| Control (miconazole) | | | | | |
| 11.0 | 1.0 | 0.6 | 1.0 | 12.5 | 1.0 |

| +FKI-1366 substance A 0.5 (2.5)* µg/ml | | | | | |
|---|---|---|---|---|---|
| 11.0 | 1.0 | 0.5 | 1.2 | 2.27 | 5.5 |

| +FKI-1366 substance A 1.0 (5.0)* µg/ml | | | | | |
|---|---|---|---|---|---|
| 7.86 | 1.4 | 0.12 | 5.0 | 1.30 | 9.6 |

| +FKI-1366 substance A 2.0 (10)* µg/ml | | | | | |
|---|---|---|---|---|---|
| <0.2 | >55 | <0.01 | >60 | 1.15 | 10.9 |

| +FKI-1366 substance B 0.5 (2.5)* µg/ml | | | | | |
|---|---|---|---|---|---|
| 6.47 | 1.7 | 0.46 | 1.4 | 6.94 | 1.8 |

| +FKI-1366 substance B 1.0 (5.0)* µg/ml | | | | | |
|---|---|---|---|---|---|
| 1.83 | 6.0 | 0.14 | 4.2 | 5.20 | 2.4 |

| +FKI-1366 substance B 2.0 (10)* µg/ml | | | | | |
|---|---|---|---|---|---|
| <0.2 | >55 | <0.01 | >60 | 3.57 | 3.5 |

| +FKI-1366 substance C 0.5 (2.5)* µg/ml | | | | | |
|---|---|---|---|---|---|
| 11.0 | 1.0 | 0.60 | 1.0 | 2.40 | 5.2 |

| +FKI-1366 substance C 1.0 (5.0)* µg/ml | | | | | |
|---|---|---|---|---|---|
| 4.4 | 2.5 | 0.55 | 1.1 | 1.56 | 8.0 |

| +FKI-1366 substance C 2.0 (10)* µg/ml | | | | | |
|---|---|---|---|---|---|
| <0.2 | >55 | 0.15 | 3.9 | 1.14 | 11.0 |

As described hereinabove, since the substance of the present invention has the enhanced action of the activity of azole antifungal agents on a strain C. albicans ATCC64548, fluconazole resistant strain C. albicans ATCC64550 and Aspergillus niger ATCC6275, the substance exhibits actions against deep-seated mycosis and other fungal infectious diseases with low concentration and within short time, and is useful for reducing frequency of appearance of resistant microorganisms. Further, usefulness for overcome the resistance will be expected. Brief explanation of drawing
Fig. 1 shows UV absorption spectrum (in methanol) of FKI-1366 substance A of the present invention.
Fig. 2 shows IR absorption spectrum (KBr tablet) of FKI-1366 substance A of the present invention.
Fig. 3 shows proton ¹H-NMR spectrum of FKI-1366 substance A of the present invention (in CDCl₃).
Fig. 4 shows ¹³C-NMR spectrum of FKI-1366 substance A of the present invention (in CDCl₃).
Fig. 5 shows UV absorption spectrum (in methanol) of FKI-1366 substance B of the present invention.
Fig. 6 shows IR absorption spectrum (KBr tablet) of FKI-1366 substance B of the present invention.
Fig. 7 shows proton ¹H-NMR spectrum of FKI-1366 substance B of the present invention (in CDCl₃).
Fig. 8 shows ¹³C-NMR spectrum of FKI-1366 substance B of the present invention (in CDCl₃).
Fig. 9 shows UV absorption spectrum (in methanol) of FKI-1366 substance C of the present invention.
Fig. 10 shows IR absorption spectrum (KBr tablet) of FKI-1366 substance C of the present invention.
Fig. 11 shows proton ¹H-NMR spectrum of FKI-1366 substance C of the present invention (in CDCl₃).
Fig. 12 shows ¹³C-NMR spectrum of FKI-1366 substance C of the present invention (in CDCl₃).

### Best mode for carrying out the invention

The present invention will be explained by mentioning example, but the present invention will not be limited within the example.

### Example

A GP medium (glucose 2.0%, yeast extract 0.2%, magnesium sulfate 7 hydrate 0.05%, polypeptone 0.5%, potassium dihydrogenphosphate 0.1% and agar 0.1%, adjusted to pH 6.0) 100 ml was added to a 500 ml Erlenmeyer flask. The flask was sealed with cotton plug and sterilized with steam. One loopful Beauveria sp. FKI-1366 FERM BP-08459 grown on the agar medium was aseptically inoculated and shake cultured at 27°C for 72 hours to prepare a seed culture medium.

The medium containing glycerol 3.0%, oat meal 2.0%, dry yeast 1.0%, potassium dihydrogenphosphate 1.0%, sodium dihydrogenphosphate 1.0% and magnesium chloride hexahydrate 0.05% (pH not adjusted) was poured into two 7.5 liter capacity jar fermenter (Marubishi Bioeng. Co., Japan) and was sterilized with steam. The seed culture medium 40 ml was inoculated aseptically and cultured at 27°C for 6 days at 200 rpm with aeration 2.5 lit./min. Acetone 5 lit. was added to the obtained whole cultured liquid, stirred well, filtered with adding Celite and concentrated in vacuo. This was again extracted with ethyl acetate and concentrated in vacuo to obtain crude substance 9.6 g.

The crude substance 4.5 g was dissolved in a small amount of methanol. The methanol solution was charged on a column of ODS resin (ODS-7515-12A, Senshu Scientific Co., Ltd.), which was equilibrated with 40% aqueous acetonitrile. Elution was performed by 40% aqueous acetonitrile 1 lit., subsequently same amount of 60% aqueous acetonitrile, 80% aqueous acetonitrile and 100% aqueous acetonitrile, respectively, and each fraction (50 ml) was collected. Fractions from No. 5 to No. 20, which were eluted with 80% aqueous acetonitrile containing FKI-1366 substance A, FKI-1366 substance B and FKI-1366 substance C, were collected. After distilled of acetonitrile, aqueous layer was extracted with ethyl acetate and dried in vacuo to obtain crude substance 1.1 g.

The crude substance 556 mg was isolated and purified by using high performance liquid chromatography. The apparatus used was PU-980 Galiver system (Nihon Bunko Inc., Japan) with the column PEGASIK ODS (20X250 mm, Senshu Scientific Co. Ltd., Japan). A mobile phase used was 85% aqueous acetonitrile and the detection was performed at UV 210 nm with elution rate 6 ml/min. Under such the condition, FKI-1366 substance A, FKI-1366 substance B and FKI-1366 substance C, which were substance having retention time 28 min., 29 min. and 42 min., respectively, were obtained separately. Each eluate was concentrated in vacuo and the remained aqueous layer was extracted with ethyl acetate to obtain FKI-1366 substance A 15.9 mg, FKI-1366 substance B 28.9 mg and FKI-1366 substance C 6.9 mg. Further, the thus obtained FKI-1366 substance A, FKI-1366 substance B and FKI-1366 substance C were fractioned and extracted again by the same condition as above to obtain finally FKI-1366 substance A 8.6 mg, FKI-1366 substance B 11.5 mg and FKI-1366 substance C 3.2 mg.

### Industrial applicability

As explained hereinabove, FKI-1366 substance A, FKI-1366 substance B and FKI-1366 substance C of the present invention have the enhancing action of the activity of azole antifungal agent against strains of C. albicans ATCC64548, fluconazole resistant C. albicans ATCC64550 and Aspergillus niger ATCC 6275. These substances act against deep-seated mycosis and other fungal infections at low concentration within short term, and are useful for reducing frequency of appearance of resistant microorganisms and for overcoming resistance, and will be expected as medicament.

## Claims

1. FKI-1366 substance A represented by the following formula [I]:

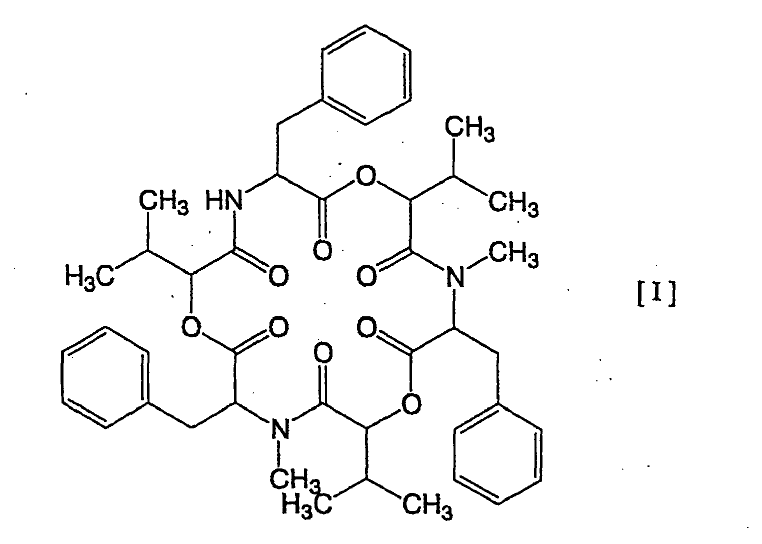

2. FKI-1366 substance B represented by the following formula [II]:

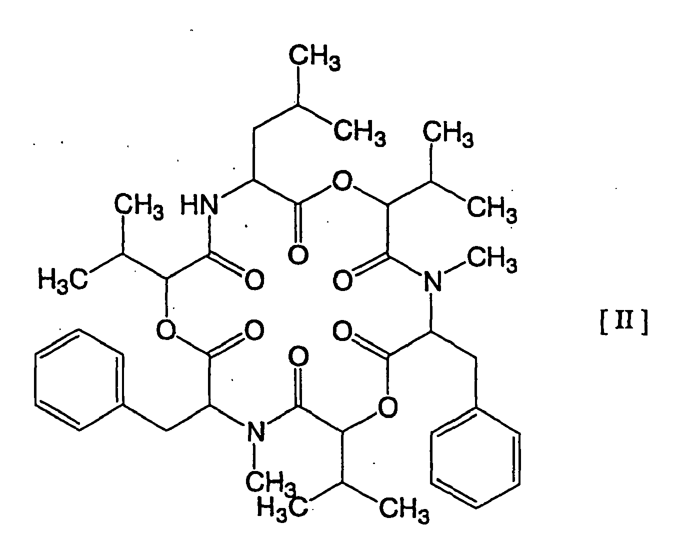

3. FKI-1366 substance C represented by the following formula [III]:

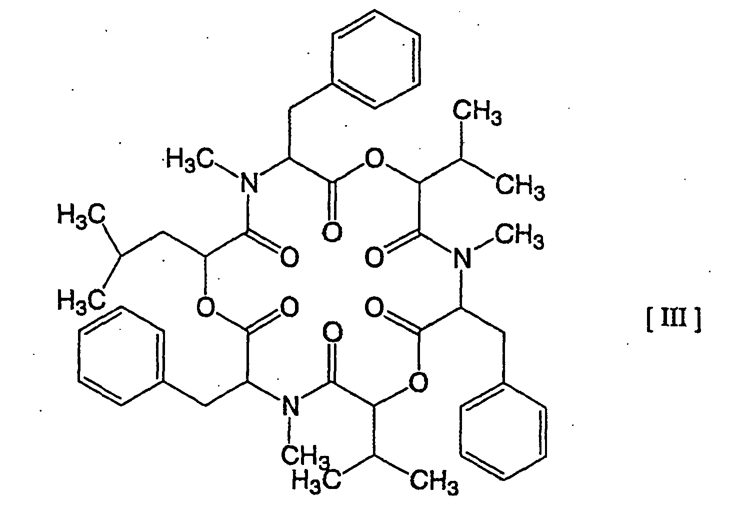

4. A crude FKI-1366 substance comprising specifically FKI-1366 substance A represented by the following formula [I]: 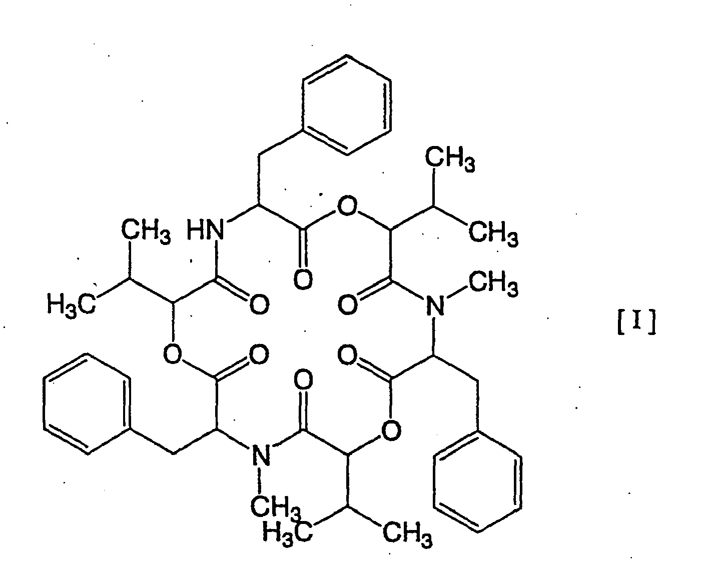 ; specifically FKI-1366 substance B represented by the following formula [II]: 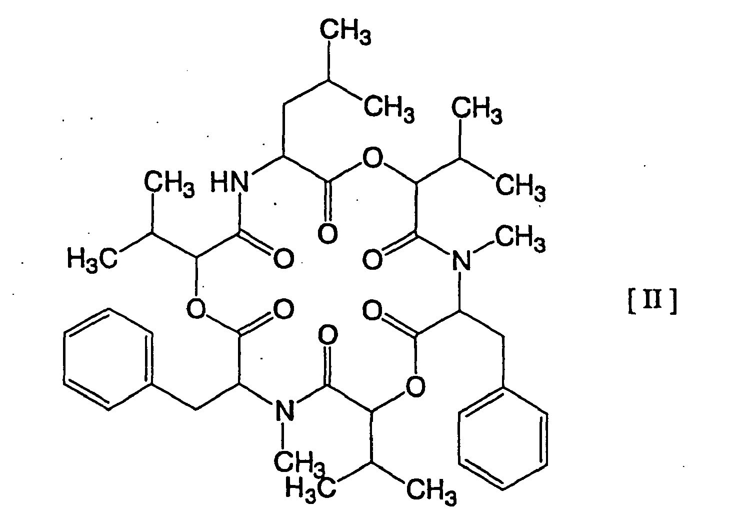 and specifically FKI-1366 substance C represented by the following formula [III]:

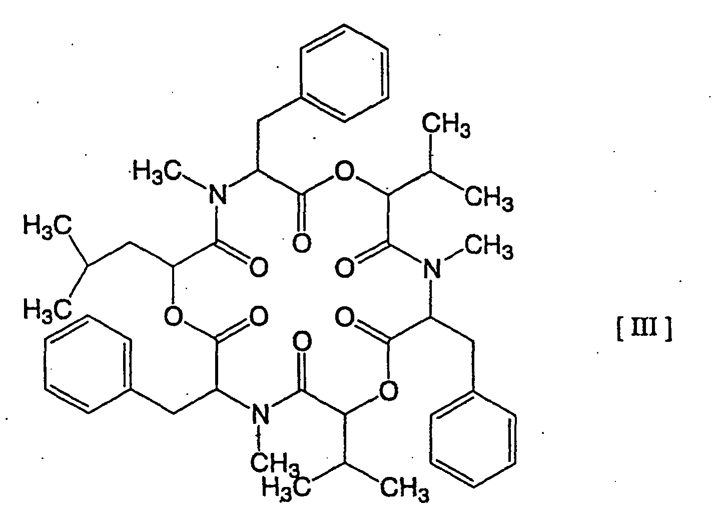

5. A process for production of FKI-1366 substance A comprising culturing a microorganism belonging to Beauveria sp. and having ability to produce FKI-1366 substance A in a medium, accumulating FKI-1366 substance A in the culture liquid medium and isolating FKI-1366 substance A from said cultured mass.

6. A process for production of FKI-1366 substance B comprising culturing a microorganism belonging to Beauveria sp. and having ability to produce FKI-1366 substance B in a medium, accumulating FKI-1366 substance B in the culture liquid medium and isolating FKI-1366 substance B from said cultured mass.

7. A process for production of FKI-1366 substance C comprising culturing a microorganism belonging to Beauveria sp. and having ability to produce FKI-1366 substance C in a medium, accumulating FKI-1366 substance C in the culture liquid medium and isolating FKI-1366 substance C from said cultured mass.

8. A process for production of crude FKI-1366 substance comprising culturing a microorganism belonging to Beauveria sp. and having ability to produce FKI-1366 substance A and/or FKI-1366 substance B and/or FKI-1366 substance C in a medium, accumulating FKI-1366 substance A and/or FKI-1366 substance B and/or FKI-1366 substance C in the culture liquid medium and isolating FKI-1366 substance A and/or FKI-1366 substance B and/or FKI-1366 substance C from said cultured mass.

9. The process for production of crude FKI-1366 substance according to claim 8 wherein the microorganism belonging to Beauveria sp. and having ability to produce FKI-1366 substance A and/or FKI-1366 substance B and/or FKI-1366 substance C is Beauveria sp. FKI-1366 FERM BP-08459.

10. Beauveria sp. FKI-1366 FERM BP-08459.

11. The microorganism belonging to Beauveria sp. and having ability to produce FKI-1366 substance A described in claim 1.

12. The microorganism belonging to Beauveria sp. and having ability to produce FKI-1366 substance B described in claim 2.

13. The microorganism belonging to Beauveria sp. and having ability to produce FKI-1366 substance C described in claim 3.

14. The microorganism having ability to produce crude FKI-1366 substance described in claim 4 wherein the microorganism is Beauveria sp. FKI-1366 FERM BP-08459 belonging to Beauveria sp. and having ability to produce FKI-1366 substance A and/or FKI-1366 substance B and/or FKI-1366 substance C.
